# FASCICULE DE BREVET EUROPEEN

(11) **EP 0 752 862 B1**
(45) Date de publication et mention de la délivrance du brevet: **25.06.2003**
(21) Numéro de dépôt: 95915222.4
(22) Date de dépôt: 29.03.1995
(51) Int. Cl.: A61K 31/715, A61P 21/00

(54) **UTILISATION DE BIOPOLYMERES POUR LE TRAITEMENT DES MUSCLES**
VERWENDUNG VON BIOPOLYMEREN ZUR BEHANDLUNG DER MUSKELN
USE OF BIOPOLYMERS FOR MUSCLE TREATMENT

(30) Priorité: 30.03.1994 FR 9403803
(43) Date de publication de la demande: 15.01.1997
(73) Titulaire: Valbiofrance, 75006 Paris (FR)
(72) Inventeur: BARRITAULT, Denis, F-75001 Paris (FR); CARUELLE, Jean-Pierre, F-94100 Saint-Maur (FR); DESGRANGES, Pascal, F-75011 Paris (FR); GAUTRON, Jean, F-94400 Vitry-sur-Seine (FR); MEDDAHI, Anne, F-94000 Créteil (FR)
(74) Mandataire: Michelet, Alain
(86) Numéro de dépôt international: FR9500398
(87) Numéro de publication internationale: WO95026736

(56) Documents cités:
- FR-A- 2 461 724
- FR-A- 2 644 066
- US-A- 4 740 594
- J. BIOMATER. SCI. POLYM. ED., vol. 3, no. 2, 1991 pages 149-154, T. AVRAMOGLOU ET AL. 'Derivatized dextran inhibition of smooth muscle cell proliferation.'
- J. BIOMATER. SCI. POLYM. ED., vol. 4, no. 5, 1993 pages 431-444, D. LETOURNEUR ET AL. 'Antiproliferative capacity of synthetic dextrans on smooth muscle cell growth: the model of derivatized dextrans as heparin-like polymers,'
- DEV. BIOL., vol. 126, no. 1, 1988 pages 19-28, E. KARDAMI ET AL. 'Heparin inhibits skeletal muscle growth in vitro.'

## Description

La présente invention a pour objet l'utilisation de polymères ou de biopolymères pour la préparation d'un médicament pour le traitement de lésions de toutes origines affectant le muscle squelettique ou le muscle cardiaque en médecine humaine ou vétérinaire et des compositions pharmaceutiques pour ce traitement.

La synthèse des polymères CMDBS (dextranes substitués par des résidus carboxyméthyle, benzylamine et sulfonate) a été décrite dans le brevet FR 2461724 ainsi que dans le brevet US 4 740 594. Certains de ces polymères miment l'héparine et peuvent être utilisés en tant que produits de remplacement de l'héparine du plasma, grâce à leurs propriétés anticoagulante et anticomplément.

Parmi l'ensemble des polymères CMDBS, certains de ces polymères miment une autre propriété de l'héparine qui consiste en une stabilisation, une protection et une potentialisation de l'activité biologique in vitro des facteurs de croissance de la famille FGF (Tardieu et coll , Journal of Cellular Physiology,1992,150 Pages 194 à 203).

Le brevet FR 2 644.066 décrit l'utilisation de certains CMDBS associés aux FGF pour la cicatrisation de la peau et de la cornée. Des expériences ont été réalisées en provoquant une blessure cutanée à l'aide d'un emporte pièce de 6 mm de diamètre chez le rat. Dans cet exemple, le CMDBS associé au FGF 2 permet d'obtenir un effet net sur la vitesse et la qualité de la réparation de la peau.

Un autre biopolymère, le dextrane sulfate a également été proposé en association avec des FGF, comme stabilisateur et protecteur, dans le brevet Japonais N° 13890. Le dextrane sulfate est par ailleurs largement utilisé dans des pommades ou crèmes cicatrisantes de la peau ainsi que dans des compositions de collyre, mais n'a aucun effet reporté à la connaissance du demandeur sur la cicatrisation et ou la régénération de lésions musculaires.

Un autre agent, le sucrose sulfate ester et son sel d'aluminium le sucralfate sont des produits décrits et utilisés seuls ou associés aux FGF comme agents de traitement des ulcères et lésions du tractus digestif (Brevet US N°3.432.489 et Brevet US N°5.202.311 ) .

Les tissus musculaires squelettique et/ou cardiaque sont particulièrement riches en facteurs de croissance et plusieurs auteurs ont décrit la présence et/ou l'action des FGFs et TGFs bêta dans et sur les cellules myoblastiques (par exemple: D.Gospodarowicz et Cheng , In Vitro Cellular and Developmental Biology 1987 23(7):pp. 507-514 ; Groux -Muscatelli B., Bassaglia Y , Barritault D. , Caruelle J.P. et Gautron J. Dev.Biol. 1990 , 142:pp380 -385 ; Johnson S. et Allen R Exp.Cell Res. 1990 , 187 : pp 250-254 ; Dayton W. et Hathaway M. Poult Sci 1991 70: pp 1815-1822 ) ainsi que leur extraction à partir de muscles squelettiques ou cardiaques (par exemple Morrow et coll. J.Clin..Invest. 1990 , 85 : pp 1616-1820 ; Padua R et E.Kardami Growth Factor , 1993 , 8 : pp 291-306 ; Parker T et Scheinder M ; Annu. Rev. Physiol. 1991 53 :pp 179-200 ; Casscells W et coll. Ann N.Y. Acad. Sci 1990, 593 :pp 148-161).

L'action cicatrisante des facteurs FGF dans des lésions du muscle cardiaque induites par création d'ischémie a été décrite (Yanagisawa-Miwa et coll. Science 1992, 257 : pp 1401-1403).

Franco (US 4.378.347) a aussi décrit l'utilisation d'aFGF pour traiter en particulier des ischémies cardiaques. Des billes de dextrane sont utilisées comme excipient dans certaines formulations décrites dans ce brevet.

L'activité de la composition est très clairement imputée au FGF.

Il ressort donc de l'analyse de l'état de la technique que des polymères avaient déjà été utilisés en association avec des facteurs de croissance.

Néanmoins, dans aucun des documents cités ces polymères ne présentent des effets en eux-mêmes, c'est-à-dire sans qu'ils soient associés à des facteurs de croissance.

En outre, l'activité d'associations polymères-facteurs n'a été décrite que sur certaines lésions d'un type bien précis de tissu, le tissu cutané.

Du fait de l'imprévisibilité des effets thérapeutiques d'une molécule donnée, il n'était pas évident que ces polymères puissent avoir un effet sur d'autres tissus que ceux de la peau.

En effet, il est bien connu que les différents tissus du corps humain ou animal présentent des spécificités tant structurelles que fonctionnelles qui rendent impossible toute prédiction quant à l'effet d'une molécule, connue pour son effet sur le tissu cutané, sur le tissu musculaire.

Ceci est d'autant plus vrai que les tissus musculaires sont très différents de par leur structure et leur origine (mésodermique) des tissus de l'épiderme et de la cornée.

De même, il est bien connu qu'il est impossible de prédire l'activité in vivo d'une molécule sur un tissu particulier à partir de résultats obtenus in vitro sur un modèle expérimental spécifique.

De manière surprenante, il a été trouvé, selon l'invention, que certains polymères ont un effet très marqué sur la vitesse de cicatrisation et de régénération des lésions des tissus musculaires squelettiques et/ou cardiaques, ainsi que sur la qualité de cette cicatrisation et/ou régénération telle que l'on peut la mesurer en étudiant par des méthodes histologiques et physiologiques le degré de maturation des fibres musculaires.

La présente invention a pour objet l'utilisation d'au moins un polymère ou d'un biopolymère, appelés HBGFPP, protégeant spécifiquement les facteurs de croissance des familles des FGF et TGF bêta de la dégradation trypsique et n'inhibant pas de manière significative la coagulation, pour la fabrication d'un médicament pour le traitement des tissus musculaires.

Un tel polymère présente particulièrement une activité anti-coagulante inférieure à 50 unités internationales par mg de polymère mesurée selon Maillet et al (Mol. Immunol, 1988, 25, 915-923). Avantageusement, il potentialise les FGF in vitro.

Préférentiellement, il n'active substantiellement pas le système du complément, c'est-à-dire qu'il possède une activité anti-complément, supérieure à 0,5 µg pour le CH5O (selon Mauzac et al., Biomaterials, 6, 61-63, 1985).

Selon la présente invention on entend par polymères toutes substances naturelles, naturelles modifiées chimiquement ou totalement synthétiques répondant à la définition donnée ci-dessus.

Ainsi il peut s'agir de :
- polymères obtenus à partir de dextranes mais modifiés par d'autres types de substitutions avec d'autres types de radicaux,
- polymères naturels autres que ceux dérivant de dextranes mais comportant des résidus osidiques (cellulose, chitine, fucanes, etc....),
- polymères obtenus par polymérisation de monomères de natures non osidiques (poly acide malique, poly acide oxalique, poly acide lactique, polystyrène, polyéthylène glycol) modifiés ou non.

Avantageusement, ledit polymère ou biopolymère est un polysaccharide qui peut être composé principalement de résidus glucose.

Un tel polysaccharide présentera préférentiellement un poids moléculaire supérieur à 10 kD et avantageusement proche de 40 kD.

Il peut aussi comprendre des résidus glucosamine et/ou d'acide uronique, particulièrement sous la forme de dimère glucosamine-acide uronique.

Des polysaccharides particulièrement préférés sont des dextranes substitués, des glycosaminoglycanes éventuellement associés à un lipide, un peptide ou un protide ou des sulfates de ces polymères.

La présente invention est en outre relative à une composition pharmaceutique contenant de ces polymères.

Les polymères et/ou biopolymères peuvent être séléctionnés à partir de substances naturelles qui peuvent ensuite être éventuellement modifiées par additions de groupements chimiques appropriés ou encore obtenus entièrement par synthèse . Ces polymères naturels, semi synthétiques ou entièrement synthétiques sont ensuite sélectionnés sur la base de leurs capacités à interagir spécifiquement avec plusieurs facteurs de croissance notamment ceux de la famille des FGF et des TGF bêta. Ils sont également sélectionnés sur leurs capacité à protéger ce ou ces facteurs contre des dégradations protéolytiques. Ces polymères seront désignés sous le sigle générique de HBGFPP (heparin binding growth factor protectors and promotors).

Deux prototypes de ces polymères ou bio polymères sont donnés comme exemples ainsi que les procédés et critères de sélection de ces polymères.

Le premier exemple de HBGFPP appartient à la famille des CMDBS qui sont des produits connus, à savoir des dextranes biospécifiques fonctionnalisés, substitués par des résidus carboxyméthyle, benzylamide et benzylamine sulfonate. Ces polymères illustrent l'obtention de HBGFPP à partir de produits naturels (dextrans) subséquemment chimiquement substitués.

Le deuxième exemple décrit la sélection de produits complètement naturels comme les protéoglycosaminoglycannes sulfates purifiés à partir d'extraits tissulaires.

Ces deux exemples illustrent les capacités de ces HBGFPP à interagir, à stabiliser, à protéger et à potentialiser les facteurs de croissances des familles FGF et TGF bêta et leur utilisation dans une composition pharmaceutique permettant une cicatrisation et une régénération des cellules musculaires squelettiques ainsi qu'une protection et cicatrisation des cellules musculaires cardiaques.

On entend , dans la présente demande, par traitement, toute opération curative ou préventive effectuée pour la prophylaxie et la cicatrisation de lésions de tissus musculaires.

Grâce à l'action des HBGFPP et notamment des CMDBS comme l'illustrent les exemples décrits ci-dessous, la réorganisation musculaire est accélérée, alors que l'architecture du muscle, c'est à dire le nombre de fibres par faisceau organisé dans le cas du muscle squelettique n'est pas modifiée de façon significative. Dans le cas du muscle cardiaque le nombre de myocytes détruits après la lésion provoquée par ischémie est très inférieur à celui que l'on peut dénombrer après traitement par les HBGFPP. (Résultats corroborés par la diminution du nombre de fibroblastes et de fibres de collagènes dans les coeurs traités au HBGFPP). Ces résultats illustrant l'effet cytoprotecteur des HBGFPP.

Un médicament ou une composition pharmaceutique selon l'invention contient une quantité efficace de HBGFPP par exemple du CMDBS associée à un ou plusieurs véhicules compatibles et pharmaceutiquement acceptables. Elle peut être également associée à des agents pharmaceutiques comme des anti-inflammatoires; ou antibactériens, et pour le muscle cardiaque, des antiarythmiques, des anticoagulants ou des agents thrombolytiques. Le véhicule peut être du sérum physiologique ou des tampons tels que le PBS contenant NaCL 0.15 Molaire ou toute autre sorte de solution compatible et non irritante pour le tissus musculaire lésé. Des formulations permettant d'obtenir des solutions pâteuses ou en gel selon les techniques courantes connues de l'homme de l'art peuvent être proposées selon le type et l'accessibilité de la lésion.

Avantageusement , un tel médicament est conçu pour être directement injectable par voie intramusculaire à une dose de 25 à 2500 ug/ml de HBGFPP comme le CMDBS dans les exemples donnés ou comme les biopolymères naturels HBGFPP tels le mesoglycanes mais la voie intraveineuse constitue une autre voie d'administration . Outre ses qualités de protecteur des facteurs de croissance "Heparin Binding" ; les HBGFPP sélectionnés selon les tests décrits ci-dessous présentent une très faible activité anticoagulante, par rapport à celle de l'héparine, trop faible pour perturber la coagulation dans le cas d'un traumatisme musculaire. Dans le cas d'une injection par voie intraveineuse la dose injectée doit être rapportée au volume sanguin de l'homme ou de l'animal ainsi traité pour que la dose de HBGFPP dans le sang soit également comprise entre 25 à 2500 µg /ml.

A titre d'exemple d'application des médicaments selon l'invention, on peut citer les atrophies et /ou dystrophies musculaires, congénitales ou acquises , et plus particulièrement :
- les maladies génétiques : comme les myopathies (Duchennes, Becker), Dystrophies des ceintures, etc...
- les maladies où il y a atonie musculaire
- les accidents médicamenteux iatrogènes (traitement à la chloroquine ou injections d'anesthésique locaux en intra musculaire ),
- les accidents traumatiques : comme ceux des sportifs élongation, déchirure, claquage, hématomes et autres, les lésions induites par des blessures ou lors d'actes chirurgicaux,
- les agressions virales ou bactériennes , par exemple le virus du polyome,
- les atrophies musculaires induites par des immobilisations ,
- les ischémies musculaires périphériques comme celles générées par les artériopathies oblitérantes périphériques des membres,

Pour le muscle cardiaque les lésions générées par diminution et suppression de l'irrigation sanguine peuvent être prévenues ou diminuées par l'utilisation de la composition de l'invention. Ainsi dans le cas d'infarctus du myocarde, l'injection dans le muscle infarcit de la composition ou son injection par voie intraveineuse permettant l'accès des HBGFPP dans le territoire cardiaque lésé.

Dans le cas de transplantation cardiaque la survie des cellules cardiaques peut être favorisée par adjonction de médicament ou de composition selon la présente invention , de même que dans le cas de cardio myoplasties .

Dans les insuffisances cardiaques générées lors des maladies héréditaires comme celle de Duchène ou de Becker ou encore dans les myocardiopathies liées aux infections virales, parasitaires ou bactériennes.

Un avantage de l'invention est que l'utilisation d'une dose unique de la composition, procure le résultat escompté, c'est à dire, la régénération complète des fibres musculaires squelettiques comme la préservation des myocytes cardiaques.

Un autre avantage concernant les lésions des tissus musculaires est que l'on favorise également la revascularisation du muscle lésé.

A titre d'exemples décrits dans les pages suivantes et concernant le muscle squelettique, une injection unique de 25 µl à 50 µg/ml de CMDBS sur le site de la blessure induit une régénération complète des fibres musculaires après 7 jours alors que dans le muscle témoin rien de tel n'est observé. Le nombre de fibres par unité de surface dans la coupe histologique transversale est 10 fois supérieur par rapport à un témoin non traité au CMDBS.

Il est à noter que ni l'héparine, ni le dextrane sulfate ne présentent des propriétés sur la régénération musculaire. Bien que ces molécules interagissent avec les FGFs et tout au moins en ce qui concerne l' héparine avec le TGF bêta , ni la sucrase, ni l'héparine, ni le dextrane sulfate ne protègent le TGF bêta contre la protéolyse induite par l'action de la trypsine ainsi que le montrent l'application des tests de criblages et de sélection des HBGFPP décrits dans les exemples ci-dessous. Ainsi en procédant à un criblage in vitro sur la base d'une protection double des FGFs et des TGFbêta contre l'action de la protéolyse induite par la trypsine il est possible de sélectionner des HBGFPP comme certains CMDBS dont ceux donnés dans ces exemples Ces mêmes critères de sélection appliqués à des biopolymères naturels comme le mésoglycan ou le sulodexide ont permis de montrer que le mesoglycane, qui présente une double activité de protection et de stabilisation pour à la fois les FGF et les TGF bêta, présente une activité favorisante pour la réparation et régénération musculaire, fait partie de la famille des HBGFPP, alors que le sulodexide qui protège les FGF contre la protéolyse induite par l'action de la trypsine n'a pas d'action protectrice significative contre l'action de la trypsine sur les TGF bêta.

L'invention sera illustrée, sans être aucunement limitée par les exemples qui suivent, dans lesquels:
La figure 1 représente la formule du CMDBS.
La figure 2 illustre la potentialisation de l'activité biologique des FGF1 (2a) et FGF2 (2b) par l'héparine, le mesoglycane et le sulodexide. La mesure de l'activité biologique est effectuée sur des cellules CCL39 par la mesure de l'augmentation de l'incorporation de thymidine tritiée en fonction de la dose de FGF1 et de FGF2 ajoutée seule ou en présence de 20 µg d'héparine , de 10 µg de mésoglycan ou de 10 µg de sulodexide.
Les figures 3 et 4 illustrent l'effet protecteur de l'héparine, du mésoglycan et du sulodexide contre une dégradation thermique du FGF1(3) et FGF2(4). Les échantillons de FGF sont incubés seuls ou en présence de 20 µg d'héparine , de 10 µg de mésoglycan ou de 10 µg de sulodexide à 20°C (a) et 37°C (b) pendant 1, 7, 15, 30 jours. La mesure de l'activité biologique présentée en abscisse correspond aux valeurs des unités de stimulation (ED5O) de l'incorporation de thymidine tritiée dans des cellules CCL39.
La figure 5a illustre l'effet protecteur de l'héparine, du mesoglycane et du sulodexide contre une dégradation protéolytique du ¹²⁵I-FGF1. La digestion protéolytique a été effectuée à 37°C et les échantillons ont été séparés par électrophorèse sur gel de polyacrylamide à 18 %. Les gels sont séchés et autoradiographiés. La première piste contient le ¹²⁵I-FGF1 seul, dans la deuxième (piste 2) le ¹²⁵I-FGF1 est incubé en présence de trypsine et d'héparine (piste 3), de mesoglycane (piste 4) ou de sulodexide (piste 5).
La figure 5b illustre l'effet protecteur de l'héparine, du mesoglycane et du sulodexide contre une dégradation protéolytique du ¹²⁵I-FGF2. La disposition des pistes est identique à celle présentée pour le ¹²⁵I-FGF1 en 5a.
Les figures 6A et 6B sont des profils d'élution sur colonne de DEAE-Trisacryl respectivement des fractions HSM (Fig. 6A) et HSS (Fig. 6B), en présence de fractions chondroïtines sulfates (CSA) pour le calibrage de la colonne.
Les figures 7, 8, 9 et 10 correspondent à des montages microphotographiques d'une hémi-coupe transversale de muscle, après 8 jours de régénération, traités ( figures 8 et 10) ou non (figures 7 et 9) par le CMDBS.
Les figures 10A à 10D représentent des électrocardiogrammes de rats non opérés (10A et 10B) et de rats opérés (11C et 11D).
Les figures 12A à 12F représentent des photographies de coupes histologiques de coeurs de rats traités par du sérum physiologique (12A et 12D), traités par du CMDBS (12B et 12E) et de rats témoins (12C et 12F).

### EXEMPLE 1

### Préparation et sélection des CMDBS

### a) Préparation des CMDBS

Les CMDBS sont des dextranes substitués par des groupements carboxyméthyle, benzylamide et benzylamide sulfonate. La méthode de synthèse des CMDBS peut être celle décrite par M.MAUZAC et J.JOSEFONVICZ dans Biomaterials 1984,5,301-304. Selon ce procédé, le carboxylméthyle dextrane (CMD) est préparé à partir de dextrane par substitution de quelques unités glycosylées avec des groupes carboxyliques sur le carbone en position 5 ou 6. Dans une deuxième étape, la benzylamide est couplée aux groupes carboxyliques pour former le carboxyméthyl-benzylamide dextrane (ou CMBD). Enfin quelques noyaux aromatiques du benzylamide sont sulfonés pour aboutir au carboxyméthyle dextrane benzylamide sulfonate ou CMDBS.

Les sels de sodium de ces dérivés sont ultraftitrés, lyophilisés et dissous dans le tampon approprié avant utilisation.

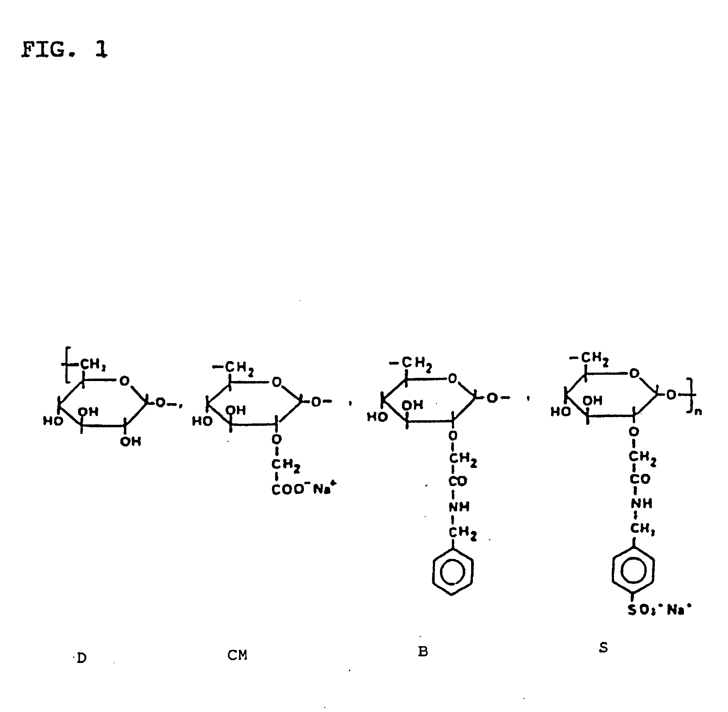

La formule générale des CMDBS est illustrée sur la figure 1.

Les CMDBS possèdent une distribution statistique des différents substituants. Les pourcentages pour chaque type de CMDBS sont déterminés par les méthodes classiques.

### b) Sélection des CMDBS

### i:Tests de protection et de stabilisation des FGFs

Lors de la synthèse des CMDBS il est possible de contrôler le taux de substitution de chacun des groupements par modification des conditions de la réaction de substitution. Le contrôle des paramètres comme la température, le temps de réaction, les concentrations relatives des constituants et le nombre de réaction de substitution etc... permettent d'obtenir un très grand nombre de polymères substitués. La substitution des hydroxyles par le carboxymethyl sur les carbones en positions 5 et 6 permet d'obtenir des taux de carboxyméthylation allant de 0 à 200% (100% pour chacun des carbones en position 5 et 6). Le groupement carboxyméthyl peut être à son tour partiellement ou totalement utilisé pour la fixation de la benzylamide. Les groupes benzylamides peuvent être partiellement ou totalement utilisés pour la sulfonation. Les dextranes substitués fonctionnalisés utilisés selon l'invention sont parmi ceux spécialement décrits dans le brevet français n°2.461.724. Outre la capacité à stabiliser et protéger les facteurs de croissance de la famille FGF comme décrit dans la publication de Tardieu et coll J.Cell.Physio.1992 150 p 194 à 203 ; et dans le brevet Français N°2.461.724; le CMDBS sélectionné doit pouvoir interagir avec au moins un membre de la famille des facteurs de croissance de la famille TGF bêta selon une méthode d'évaluation décrite ci-dessous et protéger les TGFbêta contre une protéolyse.

### ii. Evaluation des capacités d'interactions entre CMDBS et facteurs de croissance de la famille TGF bêta.

Afin de mesurer la capacité de certains CMDBS à interagir avec les membres de la famille TGF bêta et de par cette interaction protéger les TGF bêta, un test de criblage a été établi. Ce test consiste à mesurer la capacité du CMDBS sélectionné à permettre au TGF bêta de garder son activité biologique malgré un traitement protéasique.

Dans l'exemple ci-dessous le CMDBS utilisé est le lot 26.2 défini par un taux de substitution de 110% de motifs carboxyméthyles, 3,6% de motifs benzylamides et 36,5% de motifs sulfonates et possède une activité anti-coagulante de 4 UI/mg (Unités Internationales). L'activité anti-complément de ce lot est de 1,1 µg de CH5O, mesurée selon Mauzac et al (précédemment cités).

L'héparine utilisée comme témoin provient des établissements Sanofi.(Institut Choay) et présente une activité anticoagulante de 175 UI/mg

Le TFG bêtal est préparé à partir de plaquettes sanguines humaines selon le protocole décrit dans de nombreuses publications et couramment utilisés par l'homme de l'art ( par exemple dans la publication Growth Factors and their Receptors 1992 , vol 1 pages 419-472 par A. Roberts et M.Sporn édité par A. Roberts et M.Sporn et publiée par Springer Verlag Berlin. Le test d'activité biologique du TGF bêta utilisé dans cet exemple est celui de l'inhibition de croissance des cellules CCL64 (provenant de l'American Tissue Culture Collection). Cette inhibition est mesurée par la capacité du TGF bêta à inhiber l'incorporation de Thymidine tritiée d'une manière dose dépendante dans ces cellules CCL64 stimulées par le facteur de croissance FGF ou par du sérum de veau foetal selon le protocole décrit par Van Zolen dans Progress in Growth Factor Research, 1990 ,2 p131 à 152.

Le TGF bêta est utilisé à deux doses, l'une correspondant à la capacité d'inhibition de 50% de l'incorporation de Thymidine tritiée (définie comme l'unité d'activité inhibitrice) l'autre, correspondant à la capacité d'inhibition de 100%. Dans cet exemple les valeurs obtenues sont de 250 pg de TGF bêta pour obtenir l'unité d'activité d'inhibition sur les cellules CCL64 cultivées dans 1 ml de milieu de culture. Le 100% d'inhibition est obtenu avec 1ng de TGF bêta.dans 1 ml de milieu de culture.

Un échantillon de 50ng de TGF bêta dans du tampon phosphate salin contenant 0.1% de sérum albumine bovine (provenant de la Société SIGMA à Saint Louis USA) est incubé seul, ou associé soit à 5000 µg de CMDBS, soit à 5000µg d'héparine, avec ou sans 500 µg de trypsine. Le volume final de la solution incubée est ajusté à 1 ml et l'incubation est effectuée à 37°C durant un temps variable (10 minutes dans l'exemple décrit ( tableau 1).

Des échantillons d'un volume de 20 µl de chacune des réactions d'incubation sont prélevés et ajoutés aux cellules CCL64 cultivées dans des plateaux de 24 puits contenant chacun un millilitre de milieu de culture selon le protocole décrit par E.Zohlen mentionné ci dessus. Dans ces conditions la concentration finale de TGF bêta par puits est de 1ng/ml. Le tableau 1 résume les résultats obtenus dans diverses conditions et montre l'effet protecteur du CMDBS. Ainsi après 10 mn d'incubation à 37°C, 75% de l'activité biologique du TGF bêta est encore présente, alors que l'héparine qui pourtant peut se fixer au TGF bêta (Mac Caffrey et al., J. of Cell Physiology, 1992 , vol. 52, 430-440) ne protège pas le TGF bêta contre cette dégradation protéolytique (il reste moins de 20% d'activité biologique). Il est à rappeler que dans le cas des FGFs l'héparine assure une protection contre la protéolyse induite par la trypsine.(Tardieu et al., Journal of Cellular Physiology, 1992, 150: 194-203).

Il a été vérifié que le CMDBS n'avait pas de pouvoir inhibiteur sur l'activité de la trypsine (tableau 2). Ainsi,10 µg de trypsine ont été incubés soit avec un substrat (S.87 fourni par la société Serbio, Paris et utilisé selon les recommandations de ce fournisseur) ou soit avec ce substrat et un inhibiteur de la trypsine tel celui provenant du soja (comme le Soyabean trypsin inhibitor ou STI de chez Sigma) ces incubations étant faites en l'absence ou en présence de quantités variables de CMDBS (lot AM26). L'activité enzymatique de la trypsine a été mesurée par absorption spectrophotométrique du produit de transformation du S 87 en fonction du temps d'incubation.

### EXEMPLE 2:

### Sélection d'autres HBGFPP

Deux préparations commerciales de protéoglycosamino- glycane et glycosaminoglycanes ont été sélectionnées selon leurs capacités à interagir avec les facteurs de croissance de la famille du FGF ainsi qu'avec ceux de la famille du TGF bêta.

Des préparations d'héparane sulfate obtenues par fractionnement du mésoglycan et du sulodexide ont d'autre part est testées.

Le mésoglycan et le sulodexide ont été fournis par la Société Sigma Chemical Co , Saint Louis MO USA précédemment cités.

Les cellules utilisées dans cet exemple sont les cellules CC139 qui proviennent de l'American Tissue Culture Collection. Les conditions de culture et de tests de mesure d'activité biologique des FGFs sont les mêmes que celles décrites dans la publication Tardieu et coll J.Cell.Physiol. 1992. Leurs propriétés sont résumées dans le tableau 3. Les facteurs de croissance FGF utilisés sont les formes recombinantes FGF1 et FGF 2.

### a) Effet du mésoglycan et sulodexide sur l'activité biologique des FGFs in vitro.

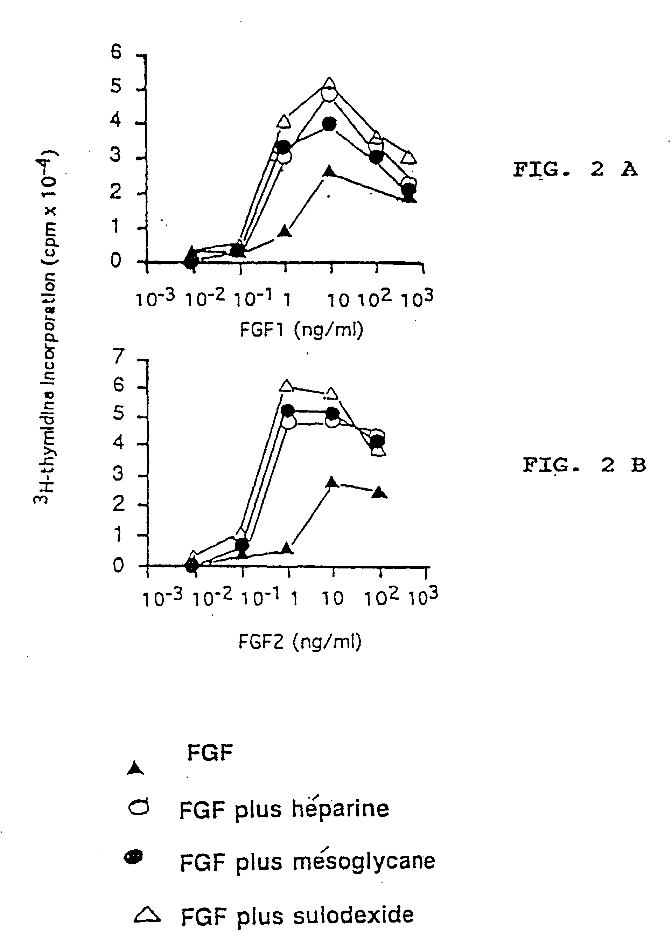

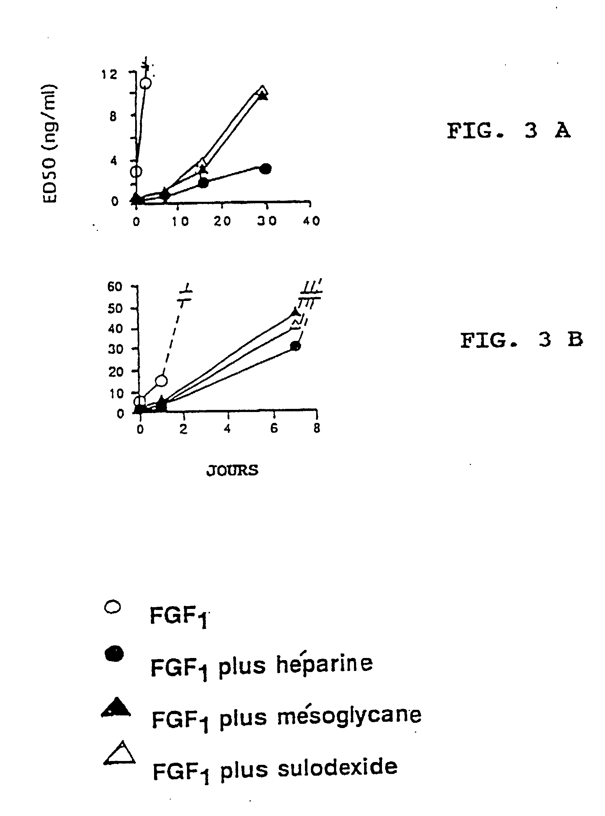

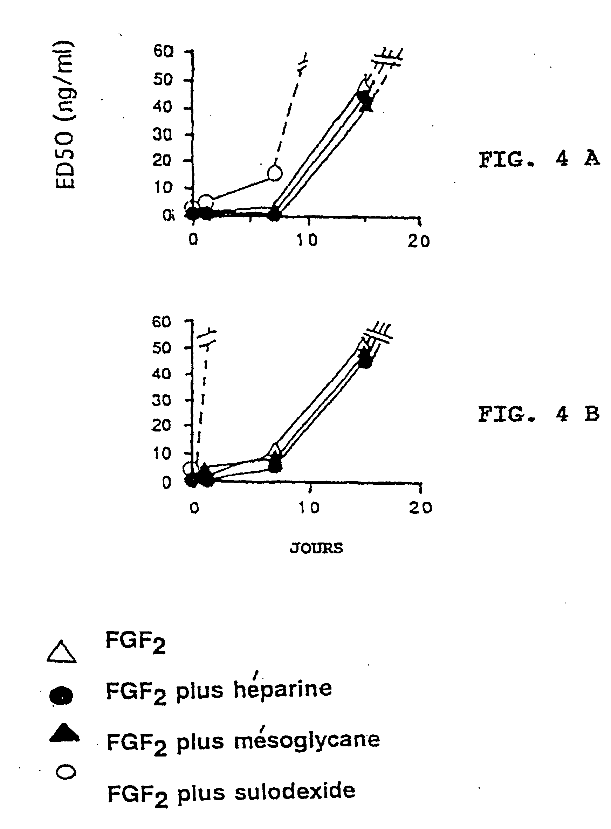

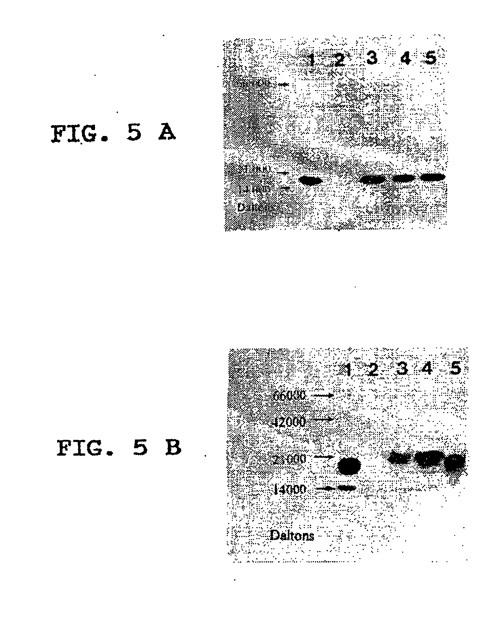

Dans ces expériences le FGF1 ou 2 est utilisé à une dose correspondant à la dose efficace (notée ED50) pour induire une stimulation de l'activité biologique de 50% de la dose induisant la stimulation maximale .L'activité biologique est mesurée par la capacité d'induire une augmentation de l'incorporation de thymidine tritiée dans les cellules selon les protocoles largement décrits dans de nombreuses publications dont celle de Tardieu et coll mentionnée précédemment et également dans le brevet français N°2 644 066. Dans cet exemple l'ED50 est de 5 ng/ml pour le FGF1 et de 3 ng/ml pour le FGF 2, valeurs mesurées expérimentalement (Figs.2a et 2b). La même expérience de stimulation en fonction de la dose de FGF est effectuée en présence de 10 µg/ml de Mesoglycan ou de Sulodexide ou 20 µg/ml d' Héparine. La figure 2 montre que dans ces conditions l'ED50 devient 0.4 ng/ml et 0,2 ng/ml respectivement pour les FGF1 et FGF2 en présence de ces doses de mésoglycan ou d'Héparine. Outre cette capacité à potentialiser l'activité biologique des FGFs les HBGFPP protègent les FGFs contre les dégradations thermiques ainsi que contre l'inactivation induite par l'action protéolytique de la trypsine.(Figs. 3 à 5). De la même manière ces HBGFPP protègent FGF1 et 2 contre une inactivation induite par l'activité protéolytique de la trypsine (Figs. Sa et 5b).

### b) Effets protecteurs du mésoglycan, sulodexine, du dextrane, du dextrane sulfate et de la sucrase vis-à-vis des TGF bêta.

Plusieurs autres composés ont été évalués: le dextrane sulfate (Sigma Chemical, de poids moléculaire 40.000, le dextrane ayant servi à la synthèse du CMDBS (également de chez Sigma) de la sucrase ou sucrose octasulfate (fournie par D. Bar Shalom, Société Bukh Medic, Danemark. Certains de ces composés ont été choisis car ils protègent et stabilisent les FGF. Ainsi, la sucrase (se confèrer au brevet US N° 520 2311) ou le dextrane sulfate (se confèrer au brevet japonais n° 1 38907/88). Le dextrane est celui qui a servi a la synthèse du CMDBS AM26.

L'expérience de protection de l'activité biologique des TGFbêta a été réalisée de la même manière qu'avec les CMDBS ainsi que décrit dans l'exemple 1 ii: Le mélange d'incubation contient 50 ng de TGF bêta ( dans 0,1 % d'albumine sérique de bovin) et de la trypsine (500 µg). Le mésoglycan ou le sulodexide ou le dextran sulfate ou le dextran ou la sucrase sont utilisés à la dose de 5000 µg.

L'activité biologique du TGFbêta est mesurée comme décrit ci-dessus après une dilution de 50 fois et en utilisant des cellules CCL64.

Les résultats sont présentés dans le tableau 4.

Ces résultats illustrent qu'à l'exception de certains CMDBS capables de répondre aux deux critères de sélection vis-à-vis des FGF et TGFbêta seul, parmi les autres composés testés, le mésoglycan présente une activité protectrice significative pour les TGFbêta.

### c) Isolement de la fraction Héparane Sulfate du Sulodexide et du Mésoglycan

Le Sulodexide et le Mésoglycan correspondent à des mélanges de plusieurs substances dont l'essentiel est constitué de différents glycosaminoglycanes (GAG).

Par une première étape de purification, il a été établi qu'un gramme de produit sec de chacun de ces deux produits contenait respectivement 874 mg pour le mésoglycan et 795 mg pour le sulodexide de GAG totaux.

Cette purification a été obtenue en soumettant ces produits solubilisés à une chromatographie échangeuse d'ions (DEAE - Trisacryl) pour enlever tous les contaminants protéiques. Les GAG totaux ont alors été purifiés en éluant le gel de DEAE avec une solution d'acétate de sodium, pH 4, contenant 1,5 M NaCl.

Après une phase de dialyse extensive contre de l'eau, 60 mg de chaque produit de GAG ont été digérés par la chondroïtinase ABC pendant une nuit à 37°C ( 1 unité par mg de GAG). Cette enzyme dégrade tous les GAG à l'exclusion des héparanes sulfates (HS). Les produits de digestion ont été soumis à une chromatographie sur tamis moléculaire (G50 Sephadex, colonne de 1,8 x 95 cm). L'élution est ensuite réalisée en tampon bicarbonate d'ammonium sous un débit de 18 ml/h. Le matériel non digéré qui correspond à des GAG de nature HS est collecté dans le volume mort d'élution de la colonne.

Les concentrations en GAG sont calculées à partir de leur contenu en acide uronique par la méthode au carbazole (Bitter T. et Muir H.M., 1962, Anal. Biochem 4, 330-334).

Ces dosages ont permis de préciser la composition suivante de chacun des produits:

| | Sulodexide | Mésoglycan |
|---|---|---|
| GAG totaux | 79 % | 87 % |
| Fraction Héparane Sulfate (HS) | 48 % | 52 % |
| Autres GAG | 31 % | 35 % |

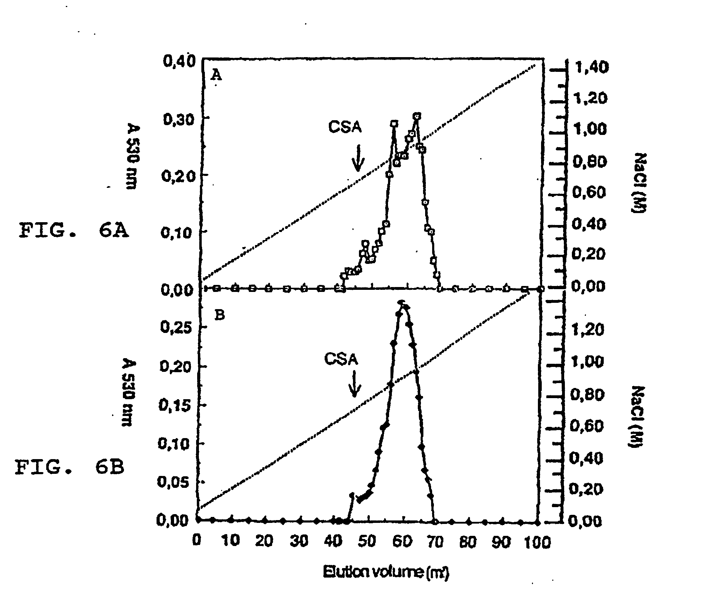

Les fractions HS de chacun de ces deux produits ont été chromatographiées à nouveau sur un gel de DEAE Trisacryl. 1 mg de chaque fraction HS, purifiée à partir du mésoglycan (Fig. 6A) ou du sulodexide ( Fig. 6B), dans 3 ml a été déposé sur une colonne équilibrée avec du tampon 0,05 M NaCI, 0,05 M TMS-Hel pH 7,5. Après un lavage de la colonne par 10 volumes du même tampon suivi d'un lavage par 10 volumes d'un tampon 0,05 M NaCl, 0,05 M d'acétate de sodium pH 4, le matériel fixé à la colonne est désorbé par un gradient salin allant de 0,05 M NaCl à 1,5 M NaCl dans le même tampon acétate. 1 ml de chaque fraction collectée a été dosé par la méthode au carbazole.

Le matériel correspondant aux constituants HS de chacun des produits d'origine présente approximativement le même profil d'élution et donc à peu près la même charge apparente. Ce maximum du pic d'élution est obtenu pour une concentration saline de 0,94 M NaCl. Une fraction définie de chondroïtine sulfate (CSA) a été soumise au même protocole en vue de calibrer la chromatographie. Cette fraction CSA qui ne contient qu'un groupe sulfate par disaccharide est élué à la force ionique de 0,72 M NaCl.

Ces résultats montrent que la fraction HS contient plus de groupements sulfates que les CSA de référence. La fraction HS présente environ deux groupes sulfates par unité dissacharidique.

Ces fractions ont été testées pour connaître leur pouvoir protecteur vis-à-vis du TGF β et du FGF en comparaison des pouvoirs établis avec les produits bruts respectifs.

### Evaluation semi-quantitative des effets protecteurs du FGF par différents polymères.

Comme décrit ci-dessus, une quantité constante de FGF radioactif est incubée dans des conditions différentes. Après autoradiographie des produits de la réaction, la quantité de FGF radioactif non dégradé est quantifiée par densitométrie. Les valeurs correspondent au pourcentage de FGF radiomarqué retrouvé par rapport à la quantité déposée en début de réaction. (Tableau 5).

Les résultats des tableaux 4 et 5 montrent que les fractions HSM et HSS issues respectivement du mésoglycan et du sulodexide présentent des effets protecteurs supérieurs à ces deux compositions et proches de 100%.

### EXEMPLE 3: Effets inhibiteurs in vitro des CMDBS et des glycosaminoglycanes sur l'activité de l'élastase leucocytaire et sur la plasmine.

Les pouvoirs d'inhibition de différents CMDBS et de leurs composés intermédiaires de leur synthèse, ont été établis pour l'élastase leucocytaire et la plasmine.

L'élastase leucocytaire purifiée a été obtenue par Elastin Products Co (Owenville, MO, USA) et la plasmine chez SIGMA.

L'inhibition des activités enzymatiques par ces différents composés est effectuée à 37°C dans un bain thermostaté. Les enzymes considérées sont mises en solution dans un tampon Tris-HCL 100 mM, pH8 pour l'élastase et pH 7,4 pour la plasmine, en présence de 0,02% d'azide de sodium et de 0,01% Triton X100 pour la plasmine. Les concentrations des substrats et celles des enzymes.sont : 0,10 mM MeO-Suc-Ala-Ala-Pro-Val-pNA (paranitroanilide) pour l'élastase à 8,3 nM et 0,20 mM dVal-Leu-dLys-pNA pour la plasmine à 77 nM. Pour chacune des conditions est établi l'IC50.

Le tableau 6 donne les résultats obtenus dans lesquels, le lot AM6 correspond à un dextrane T40 de 40 000 kD. Le lot EM5 correspond à un dextrane T10 de 10 000 kD. Les produits intermédiaires de synthèse sont répertoriés d'après les sigles désignés ci-dessus indexés d'un numéro qui précise le nombre de chacune des réactions de substitution.

Les valeurs des IC50 démontrent que les CMDBS ont des effets inhibiteurs de type hyperbolique non compétitif sur l'activité de l'élastase leucocytaire comparables à ceux de l'héparine, l'un des meilleurs inhibiteurs de cette activité (Ki de l'ordre de 1 nM). Les CMDBS exercent de plus et à l'inverse de l'héparine des effets inhibiteurs sur la plasmine.

Il ressort en outre du tableau 6 que les effets inhibiteurs des fractions HSM et HSS sont supérieurs à ceux du mésoglycan et du sulodexide, respectivement.

### Exemple 4 .

### Régénération de muscles squelettiques après écrasement simple.

### PROTOCOLE EXPERIMENTAL

L'expérimentation a été effectuée sur sept rats Wistar âgés de deux mois et demi et pesant trois cent grammes .

Après anesthésie par l'éther , les muscles EDL (muscles des pattes postérieures de rat) ont été dégagés de la loge antérieure de la jambe et lésés mécaniquement par application d'une pression constante sur toute la longueur du muscle à l'aide d'une pince de Péan. La pression est maintenue pendant quinze secondes , la pince étant fermée au deuxième cran. L'homogénéité de la lésion sur la totalité du muscle a été contrôlée en suivant son infarcissement pendant trente secondes . Le muscle a été ensuite replacé dans sa loge et la peau suturée par du fil de lin.

Les muscles EDL ont reçu une injection unique de 200 µl de CMDBS ou de sulfate de dextrane (DS) à 50 µ/ml dilués dans le PBS sans calcium ni magnésium . Les témoins de régénération ont reçu une injection de la même quantité de PBS seul . Selon les expériences , l'injection a été pratiquée avant ou après la lésion du muscle . Les 200 µl ont été injectés en une minute et la diffusion du produit a été assurée en laissant en place le muscle pendant deux minutes avant de le replacer dans sa loge . Cinq muscles EDL ont été traités par le CMDBS et deux par le sulfate de dextrane .

Les muscles traités et témoins ont été prélevés après huit jours de régénération, puis congelés immédiatement à -150°C dans l'isopentane . Des coupes transversales à congélation de 10 µm d'épaisseur ont été effectuées dans la région médiane du muscle . Les coupes séchées ont été colorées par le trichrome de Gomori.

L'analyse morphométrique du nombre de fibres et de leur diamètre a été effectuée sur des montages micrographiques correspondant à une hémi-coupe transversale de muscle.

### RESULTATS:

Lors du prélèvement des muscles après 8 jours de régénération, l'examen macroscopique montre une différence d'aspect entre ceux qui ont été traités par le CMDBS et ceux qui n'ont reçu qu'une injection de PBS. Les muscles traités ont un aspect rouge foncé alors que les témoins ont une coloration beaucoup plus claire . Cette différence suggère dans le premier cas, une vascularisation plus riche et un taux de myoglobine plus élevé . Par ailleurs, le diamètre des muscles injectés par le CMDBS est nettement augmenté ( voir tableau 7 ) ; ils occupent toute la loge antérieure de la jambe et ils refoulent le muscle tibial antérieur vers l'extérieur.

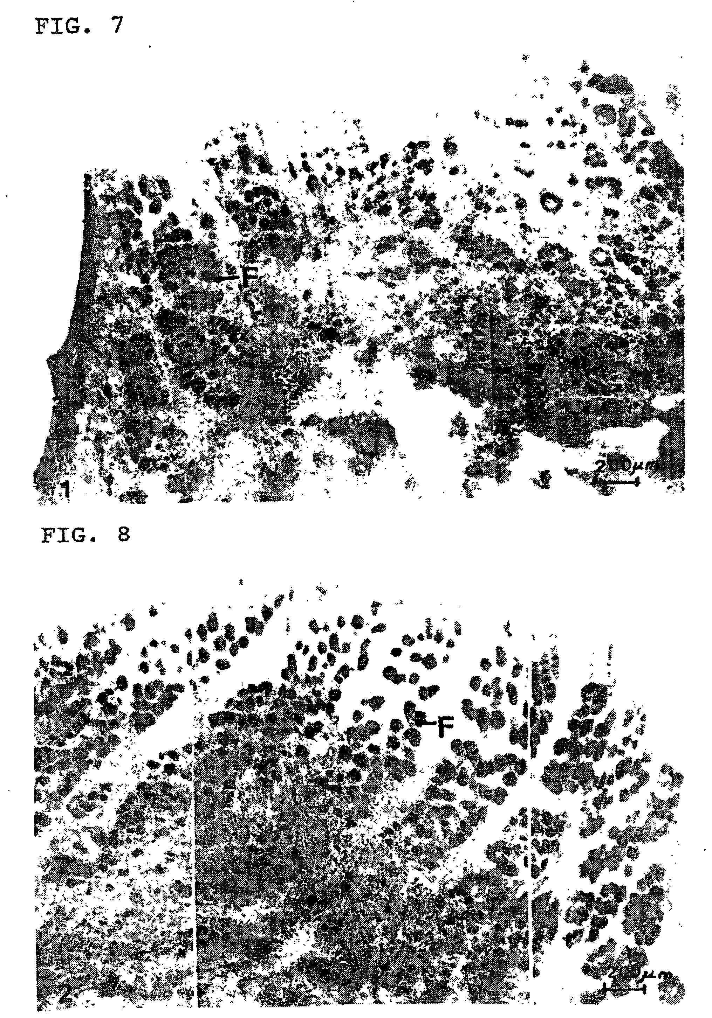

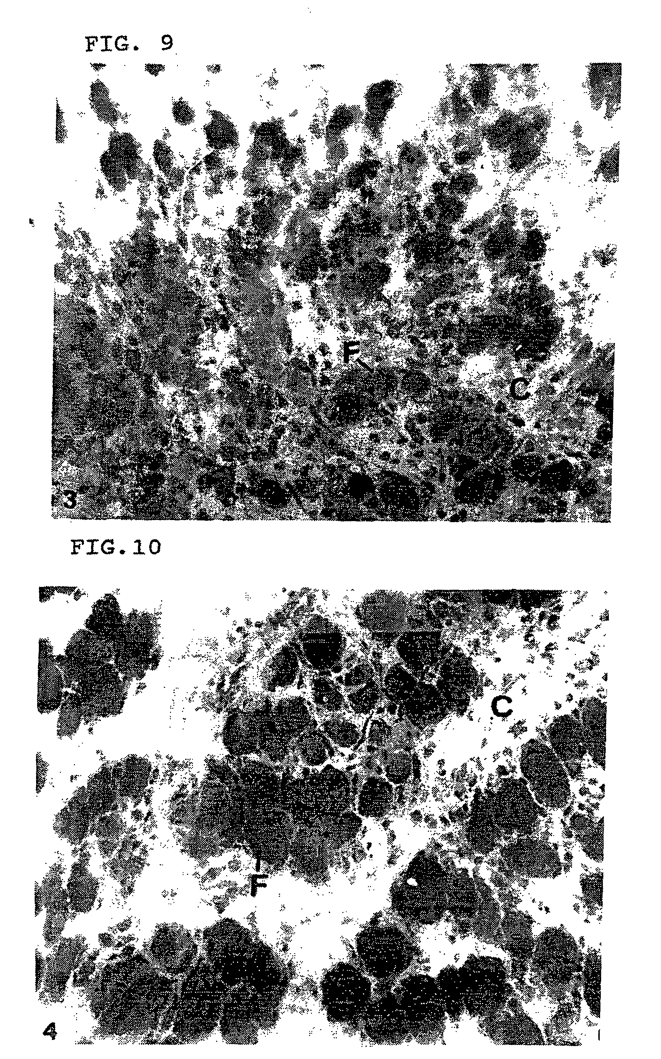

Les figures 7 à 10 correspondent à des montages microphotographiques d'une hémi-coupe transversale de muscle EDL après 8 jours de régénération traités ou non par le CMDBS. Le muscle traité (figure 8) présente un plus grand nombre de fibres régénérées (F) et d'un diamètre plus élevé que le muscle non traité (figure 7 ) . L'injection unique de CMDBS au moment de la lésion induit également une meilleure reconstruction du muscle : les faisceaux musculaires , séparés par le conjonctif du périmysium (C) , sont bien différenciés dès le 8ème jour (figure 9 non traité et figure 10 traité au CMDBS). Dans la régénération normale , ce stade de re- construction n'est atteint qu'après trois semaines . L'examen histologique des coupes montre également une plus grande vascularisation et un degré de réinnervation plus élevé.

Les résultats sont identiques que l'injection du muscle EDL ait été pratiquée avant ou après la lésion.

Les données du tableau 7 montrent que l'effet du CMDBS est très important sur la vitesse de régénération ( nombre de fibres régénérées en 8 jours ) et sur leur degré de maturation (diamètre moyen des fibres ) . La réorganisation du muscle est également nettement accélérée ( nombre de faisceau organisés). Par contre, l'architecture du muscle ( nombre de fibres par faisceau) n'est pas modifiée de façon significative .

L'injection de sulfate de dextrane, dans les mêmes conditions que celles du CMDBS, n'induit aucune modification qualitative et quantitative de la régénération musculaire .

### EXEMPLE 5:

### Régénération d'un muscle squelettique après désinnervation puis écrasement.

### Etude comparative de différents HBGFPP et autres glycosaminoglycanes non HBGFPP.

L'expérimentation est effectuée sur 18 rats Wistar mâles âgés de deux mois et demi et pesant environ 300 grammes.

Le protocole est rigourement identique à celui de l'exemple 4 avec cependant préalablement à l'écrasement du muscle, section du nerf moteur à l'entrée du muscle.

Les substances sont en solution de tampon phosphate saline (PBS).

L'injection a lieu après déinnervation et écrasement à l'aide d'une microseringue de 50 µl munie d'une aiguille flexible de 60 mm de longueur et de 0,4 mm de diamètre. Deux injections sont effectuées à 2 minutes d'intervalles. Le volume injecté est donc de 100 µl. La sucrase (glucose octyl sulfate) a été fournie gracieusement par D. Bar Shalom Bulk Medic Danemark.

Après sacrifice de l'animal, les muscles EDL de la patte traitée et de la patte controlatérale sont pesés. Les variations individuelles du poids des muscles normaux d'animaux de même âge pouvant atteindre 20%, les résultats ont été standardisés en calculant le rapport du muscle traité à son témoin intact controlatéral.

Ce ratio pour les muscles traités par le PBS seul a été considéré comme 100 %. Le degré de régénération des muscles traités par les différentes substances a été calculé en % du muscle injecté par le PBS . Les résultats sont exprimés dans le tableau 8.

Ces résultats démontrent l'effet spécifique des HBGFPP dans la régénération musculaire. En effet, seuls certains CMDBS et le mésoglycan, qui montrent un effet protecteur et promoteur à la fois pour les FGF et les TGFbêta, induisent une régénération significative ( 190% et 133 % respectivement).

### Exemple 6:

### Cicatrisation du muscle cardiaque

Les expériences réalisées lors de la cicatrisation du myocarde infarcie sont effectuées chez le rat.

Le rat représente un bon modèle expérimental par rapport à d'autres espèces animales car, comme pour l'homme , il n'existe pas de circulation collatérale entre les coronaires droite et gauche.

### protocole expérimental :

Des rats Wistar (Wi/Wi Ico, IFFA CREDO, France), de sexe mâle, pesant 350 g, sont anesthésiés au penthobarbital sodique par voie intra-péritonéale.

Les rats sont rasés au niveau du thorax et du cou, et un électrocardiogramme (ECG) est pratiqué en préopératoire.

Une trachéotomie permet de mettre le rat sous ventilation assistée, et de lutter contre le pneumothorax per opératoire et de diminuer la mortalité per et post opératoire.

Après installation du rat en décubitus latéral droit et mise en place d'un petit billot sous le thorax, une thoracoto- mie latérale gauche (à un travers de doigt au-dessus des côtes flottantes ce qui correspond au 4-5ème espace intercostal gauche) est pratiquée. Le coeur est mis à jour et le péricarde est incisé, le ventricule gauche est repéré et l'artère coronaire gauche est ligaturée à son origine avec un fil PROLENE 6/0. Une minute après, la zone infarcie est visible et l'enregistrement de l'activité électrique cardiaque permet de visualiser les ondes de Pardie témoin de la réalisation de l'infarctus.

On injecte en plein milieu de la région infarcie, en un seul point, à l'aide d'une seringue de Hamilton, 10µl d'une solution à 50 µg/ml de CMDBS préparée dans du sérum physiologique ou 10 µl de cette même solution ne contenant pas de CMDBS.

Le thorax est alors refermé, le pneumothorax est aspiré, et la ventilation assistée est laissée en place jusqu'au réveil complet de l'animal. Un dernier ECG est réalisé.

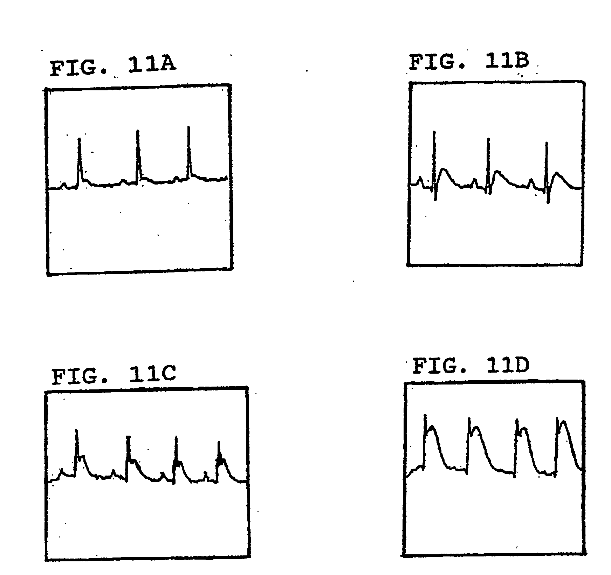

Les électrocardiogrammes (ECG) des rats avant et après infarctus au niveau de la dérivation sont représentés sur la figure 11. Les traces A et B montrent deux types d'électrocardiogramme qui peuvent être rencontrés chez les rats non opérés. Les traces C et D sont les ECG de ces mêmes rats en postopératoire. Il existe un sus décalage très net du segment ST signant l'infarctus.

Les rats retenus pour l'expérimentation, seront ceux qui présentent au moment de la fermeture du thorax, les signes électriques typiques de l'infarctus du myocarde, les autres animaux seront éliminés de l'étude.

Après examen des ECG, 33 rats seront sacrifiés aux jours 7, 15 et 30 post opératoire et un ECG sera réalisé avant l'euthanasie. Une étude histologique est pratiquée sur les coeurs infarcies.

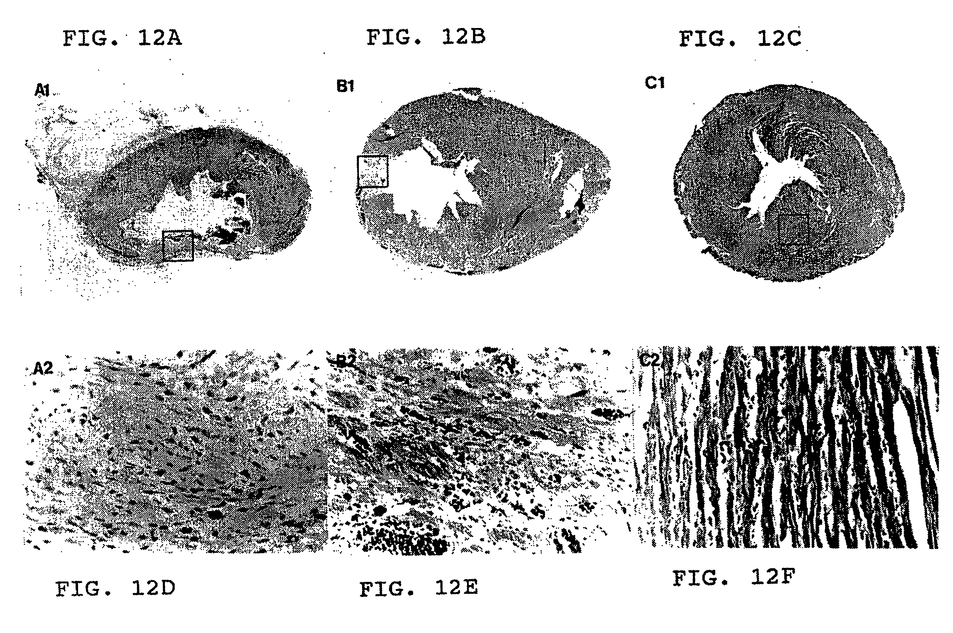

Les résultats histologiques sont présentés sur la Fig.12 qui illustre l'étude histologique au 15ème jour postopératoire des coeurs de rats infarctés par ligature de l'artère coronaire gauche et coloration au trichrome de Masson.

Figures 12A et 12D (injection de 10µl de sérum physiologique au milieu de la zone infarcie). On constate l'existence d'un infarctus sous endocardique siégeant au niveau de la paroi du ventricule gauche (12A ). Celle-ci est atrophiée et il existe une importante réaction inflammatoire en périphérie ainsi qu'une fibrose intense. La figure 12D (x25) visualise le milieu de l'infarctus. On constate l'existence d'une atrophie importante marquée par l'apparition d'une fibrose et la disparition totale des fibres myocardiques, il existe quelques cellules inflammatoires.

Figures 12B et 12E : Traitement de l'infarctus avec du CMDBS (10 µl d'une solution à 50 µg/ml sont injectés dans la zone infarcie). L'infarctus est également sous endocardique et siège au niveau du ventricule gauche, la zone atrophiée est moins étendue et il existe au niveau des fibres myocardiques intactes (12B). La figure 12E (x25) montre des zones vasculaires nombreuses, des fibres myocardiques apparemment intactes dans un environnement fibreux

Figures 12C et 12F: photographies d'un coeur témoin.

Il parait évident que la taille du ventricule gauche est conservée au niveau de la paroi des coeurs traités au CMDBS, les coeurs de la série témoin présentent des parois plus fines et une zone infarcie plus étendue. Il existe au niveau des coeurs infarcis traités au CMDBS des zones de régénération du myocarde.

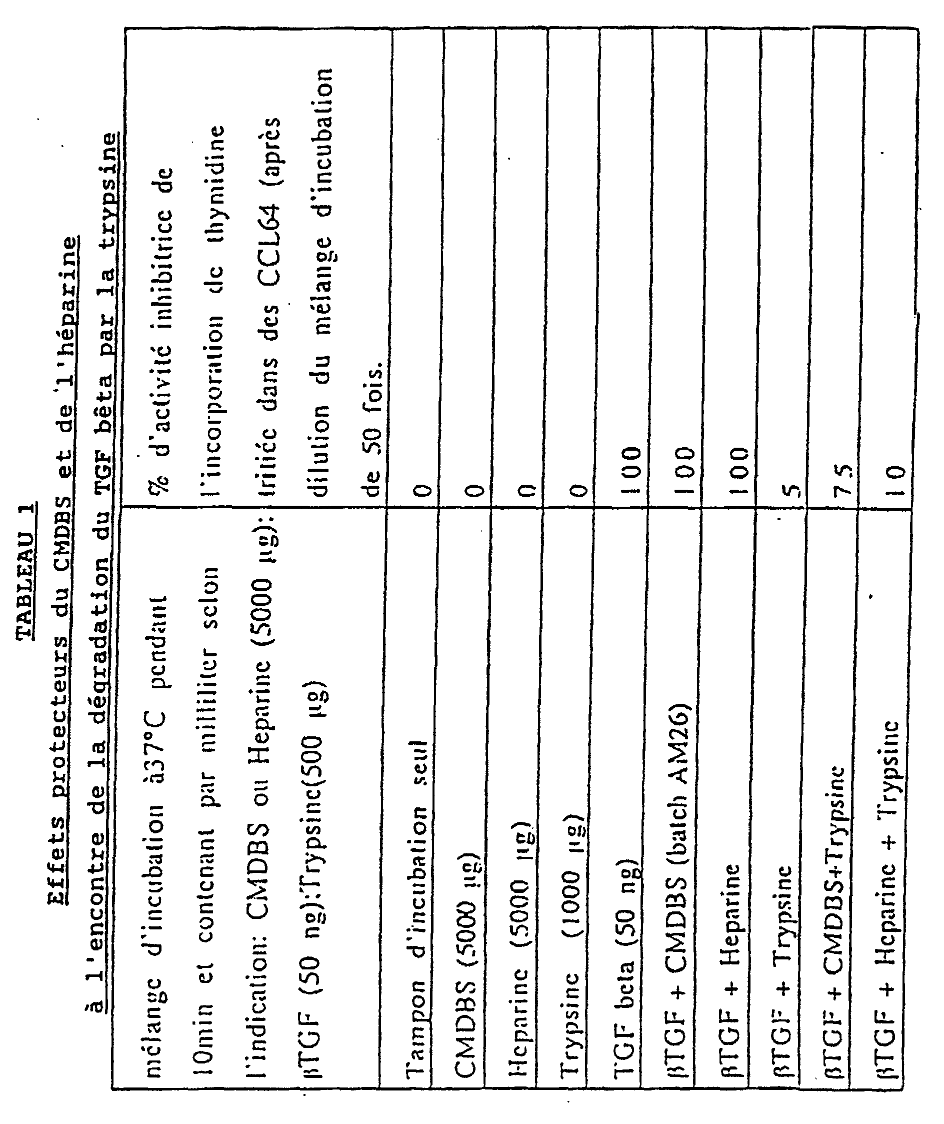

**TABLEAU 2**

| Effet non inhibiteur du CMDBS vis-à-vis de la trypsine | |
|---|---|
| Trypsine (10 µg/ml) + S87 | 100 |
| Trypsine + S87 + 5µg/ml CMDBS | 100 |
| Trypsine + S87 + 50 µg/ml CMDBS | 100 |
| Trypsine + S87 + 500 µg/ml CMDBS | 100 |
| Trypsine + S87 + STB1 | 0 |

**TABLEAU 3 :**

| Origine , activité anticoagulante et composition partielle du mésoglycan et du sulodexide (informations du fournisseur | | |
|---|---|---|
| | Sulodexide | Mésoglycan |
| Origine | duodénum de porc | aorte |
| Activité anticoagulante | 50-70 IU/mg | < 50 IU /mg |

| Composition chimique | | |
|---|---|---|
| Dermatane sulfate | 20-35% | 25 - 60% |
| Chondroitine Sulfate | 2-7% | 3-15% |
| Héparane sulfate | + | + |

**TABLEAU 4**

| Protection du TGFbêta par divers polymères | |
|---|---|
| TCF bêta | 100% |
| TGF bêta + trypsine | 0 % |
| TGF bêta + mésoglycan | 100 % |
| TGF bêta + mésoglycan + trypsine | 50 % |
| TGF β + HSM | 100 % |
| TGF β + HSM + trypsine | 75 % |
| TGF beta + sulodexide | 100 % |
| TGF beta + sulodexide + trypsine | 20 % |
| TGF β + HSS | 100 % |
| TGF β + HSS + trypsine | 45 % |
| TGF bêta + Dextrane | 100 % |
| TGF bêta + Dextrane + trypsine | 0 % |
| TGF bêta + Dextrane Sulfate | 100 % |
| TGF bêta + Dextrane Sulfate + trypsine | 0 % |
| TGF bêta + Sucrase | 100 % |
| TGF bêta + Sucrase + trypsine | 0 % |
| HSM = Héparanes Sulfates purifiés à partir au Mésoglycan | |
| HSS = Héparanes Sulfates purifiés à partir de Sulodexide | |

**TABLEAU 5**

| Protection du FGF par divers polymères | |
|---|---|
| | PROTECTION EN % |
| FGF seul | 100 |
| FGF + Trypsine | 0 |
| FGF + Trypsine + Héparine | 100 |
| FGF + Trypsine + Mesoglycan | 75 |
| FGF + Trypsine + Sulodexide | 70 |
| FGF + Trypsine + Mesoglycan traité | 0 |

| Heparinase | |
|---|---|
| FGF + Trypsine + Sulodexide traité | 0 |
| Héparinase | |
| FGF + Trypsine + Héparine Traité | 0 |

| Héparinase | |
|---|---|
| FGF + HSM + Trypsine | 95 |
| FGF + HSS + Trypsine | 90 |

**TABLEAU 6**

| Inhibition des activités de l'élastase et de la plasmine | | |
|---|---|---|
| Composés testés | Elastase Leucocytaire | Plasmine |
| | IC₅₀ en µg/ml | IC₅₀ en µg/ml |
| CMDBS lot AM6 | 2,2 | 1,5 |
| T40 | > 100 | > 100 |
| CMDBS lot EM5 | 10 | 7 |
| T10 CMD2B | 50 | 53 |
| T10 5CMD1B | > 100 | > 100 |
| T10 3CMD | > 100 | > 100 |
| T10 | > 100 | > 100 |
| Mesoglycan | 72 | 65 |
| HS Mesoglycan | 20 | 22 |
| Sulodexide | 79 | 75 |
| HS Sulodexide | 25 | 20 |
| Héparine | 1,8 | |
| Lipo-héparine | | 0,5 |
| HSM = Héparanes Sulfates purifiés à partir du Mésoglycan | | |
| HSS = Héparanes Sulfates purifiés à partir de Sulodexide | | |

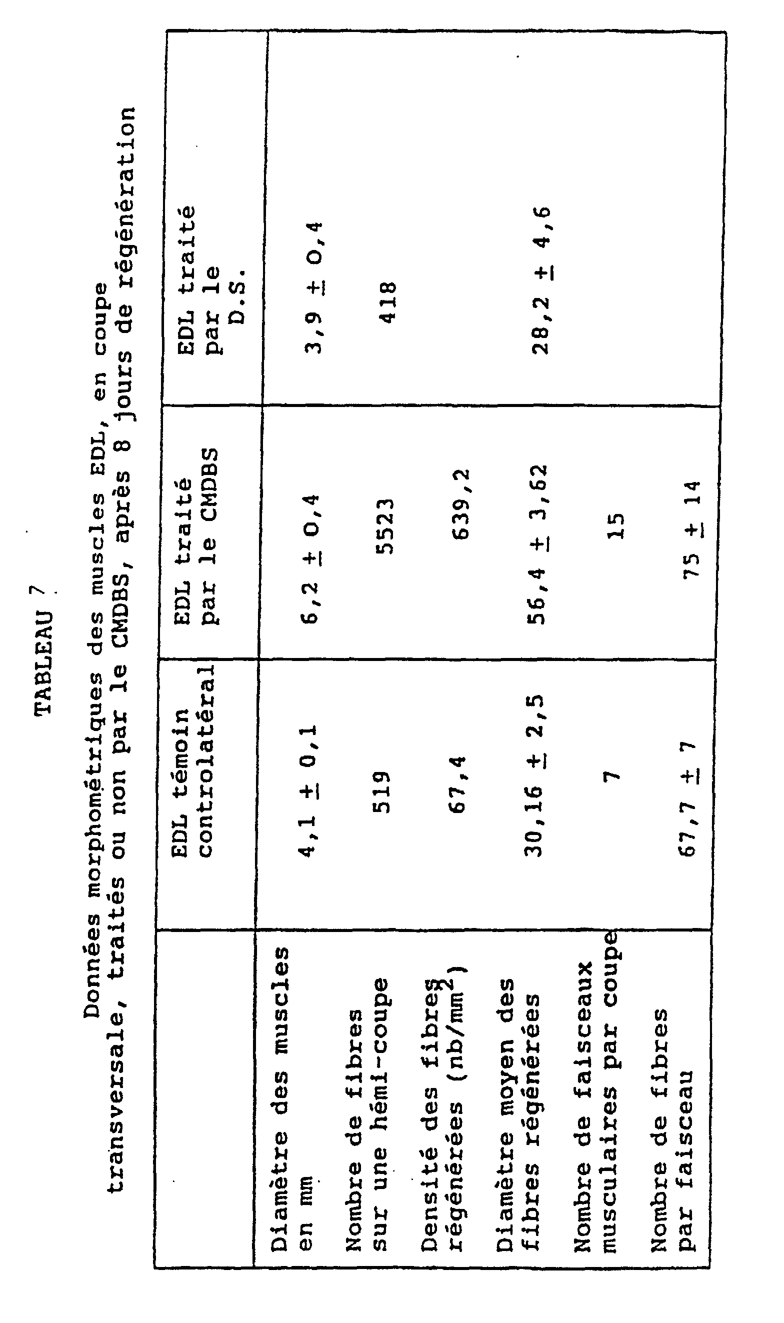

**TABLEAU 8**

| Effets de différents polymères sur la régénération de muscles désinnervés | | |
|---|---|---|
| Substances:poids injecté | Ratio du régénéré régénéré/controlatéral intact | % ratio traité/ratio dégénéré |
| PBS | 0.705 | 100 |
| CMDBS (10 µg) | 1.342 | 190 |
| Sucrase (10 µg) | 0.620 | 87 |
| Héparine (10 µg) | 0.590 | 86 |
| Sulodexide (10 µg) | 0.638 | 90.7 |
| Mésoglycane (10 µg) | 0.940 | 133 |

## Revendications

1. Utilisation d'au moins un polymère ou d'un biopolymère, appelés HBGFPP, protégeant spécifiquement les facteurs de croissance des familles des FGF et TGF bêta de la dégradation trypsique et n'inhibant pas de manière significative la coagulation, pour la fabrication d'un médicament pour la prophylaxie et la cicatrisation de lésions des tissus musculaires squelettiques ou cardiaques.

2. Utilisation selon la revendication 1, **caractérisée en ce que** le polymère ou biopolymère présente une activité anti-coagulante inférieure à 50 unités internationales par mg de polymère.

3. Utilisation selon l'une des revendications 1 et 2, **caractérisée en ce que** ledit polymère n'active substantiellement pas le système du complément

4. Utilisation selon l'une des revendications 1 à 3, **caractérisée en ce que** ledit polymère potentialise in vitro les FGF.

5. Utilisation selon l'une des revendications 1 à 4, **caractérisée en ce que** ledit polymère inhibe substantiellement les activités protéasiques de l'élastase et/ou de la plasmine.

6. Utilisation selon l'une des revendications 1 à 5, **caractérisée en ce que** ledit polymère ou biopolymère est un polysaccharide.

7. Utilisation selon la revendication 6 , **caractérisée en ce que** ledit polysaccharide est principalement composé de résidus glucose.

8. Utilisation selon la revendication 6, **caractérisée en ce que** le polysaccharide comprend des résidus glucosamine et/ou d'acide uronique.

9. Utilisation selon la revendication 8, **caractérisée en ce que** le polysaccharide comprend des dimères glucosamine-acide uronique.

10. Utilisation selon l'une des revendications 8 et 9, **caractérisée en ce que** ledit polysaccharide est un protéoglycosaminoglycane ou un glycosaminoglycane, ou un sulfate d'un de ces composés.

11. Utilisation selon l'une des revendications 1 à 6, **caractérisée en ce que** ledit polysaccharide est un dextrane substitué.

12. Utilisation selon la revendication 11, **caractérisée en ce que** ledit polysaccharide est un CMDBS.

13. Utilisation selon l'une des revendications 1 à 5, **caractérisée en ce que** ledit polymère est de nature non-osidique.

14. Utilisation ou composition selon l'une des revendications 1 à 13, **caractérisée en ce que** le tissu musculaire est celui des muscles cardiaques ou squelettiques.

15. Composition pharmaceutique pour la cicatrisation des tissus musculaires squelettiques ou cardiaques contenant au moins un polymère tel que défini dans l'une des révendications 1 à 11 en association avec au moins un excipient pharmacologiquement acceptable, à l'exception d'une composition pharmaceutique contenant une association d'héparine et de sulodexide.

## Patentansprüche

1. Verwendung mindestens eines Polymers oder eines Biopolymers mit der Bezeichnung HBGFPP, das insbesondere die Wachstumsfaktoren der FGFund der β-TGF-Familie vor dem Trypsinabbau schützt und die Gerinnung nicht signifikant hemmt, zur Herstellung eines Medikaments zur Prophylaxe und zur Heilung von Läsionen von Skelett- oder Herzmuskelgeweben.

2. Verwendung nach Anspruch 1, **dadurch gekennzeichnet, dass** das Polymer oder Biopolymer eine gerinnungshemmende Aktivität von weniger als 50 internationalen Einheiten pro mg Polymer aufweist.

3. Verwendung nach einem der Ansprüche 1 und 2, **dadurch gekennzeichnet, dass** das Polymer das restliche System im wesentlichen nicht aktiviert.

4. Verwendung nach einem der Ansprüche 1 bis 3, **dadurch gekennzeichnet, dass** das Polymer die FGF in vitro potenziert.

5. Verwendung nach einem der Ansprüche 1 bis 4, **dadurch gekennzeichnet, dass** das Polymer die Proteasenwirkungen von Elastase und/oder von Plasmin im wesentlichen hemmt.

6. Verwendung nach einem der Ansprüche 1 bis 5, **dadurch gekennzeichnet, dass** das Polymer oder Biopolymer ein Polysaccharid ist.

7. Verwendung nach Anspruch 6, **dadurch gekennzeichnet, dass** das Polysaccharid hauptsächlich aus Glucoseresten besteht.

8. Verwendung nach Anspruch 6, **dadurch gekennzeichnet, dass** das Polysaccharid Glucosamin- und/oder Uronsäurereste umfasst.

9. Verwendung nach Anspruch 8, **dadurch gekennzeichnet, dass** das Polysaccharid Glucosamin-Uronsäure-Dimere umfasst.

10. Verwendung nach einem der Ansprüche 8 und 9, **dadurch gekennzeichnet, dass** es sich bei dem Polysaccharid um ein Proteoglycosaminglycan oder ein Glykosaminoglycan oder ein Sulfat einer dieser Verbindungen handelt.

11. Verwendung nach einem der Ansprüche 1 bis 6, **dadurch gekennzeichnet, dass** es sich bei dem Polysaccharid um ein substituiertes Dextran handelt.

12. Verwendung nach Anspruch 11, **dadurch gekennzeichnet, dass** es sich bei dem Polysaccharid um ein CMDBS handelt.

13. Verwendung nach einem der Ansprüche 1 bis 5, **dadurch gekennzeichnet, dass** das Polymer eine Nicht-Glycosid-Beschaffenheit hat.

14. Verwendung oder Zusammensetzung nach einem der Ansprüche 1 bis 13, **dadurch gekennzeichnet, dass** es sich bei dem Muskelgewebe um dasjenige von Herz- oder Skelettmuskeln handelt.

15. Pharmazeutische Zusammensetzung zur Heilung von Skelett- oder Herzmuskelgeweben, enthaltend mindestens ein Polymer gemäß der Definition in einem der Ansprüche 1 bis 11 in Verbindung mit mindestens einem pharmazeutisch annehmbaren Trägermittel mit Ausnahme einer pharmazeutischen Zusammensetzung, die eine Kombination von Heparin und Sulodexid enthält.

## Claims

1. Use of at least one polymer or one biopolymer, called HBGFPP, specifically protecting the growth factors of FGF and beta TGF families from tryptic degradation and not significantly inhibiting coagulation, in the manufacture of a drug for the prophylaxis and lesion healing of skeletal or cardiac muscular tissues.

2. Use according to claim 1, **characterized in that** the polymer or biopolymer has an anticoagulant activity of less than 50 international units per mg of polymer.

3. Use according to one of claims 1 and 2, **characterized in that** the said polymer does not substantially activate the complement system.

4. Use according to one of claims 1 to 3, **characterized in that** the said polymer potentialises the FGFs in vitro.

5. Use according to one of claims 1 to 4, **characterized in that** the said polymer substantially inhibits the proteasic activities of elastase and/or plasmin.

6. Use according to one of claims 1 to 5, **characterized in that** the said polymer or biopolymer is a polysaccharide.

7. Use according to claim 6, **characterized in that** the said polysaccharide is mainly made of glucose residues.

8. Use according to claim 6, **characterized in that** the polysaccharide comprises glucosamine and/or uronic acid residues.

9. Use according to claim 8, **characterized in that** the polysaccharide comprises glucosamine-uronic acid dimers.

10. Use according to one of claims 8 and 9, **characterized in that** the said polysaccharide is a proteoglycosaminoglycan or a glycosaminoglycan, or a sulfate of one of these compounds.

11. Use according to one of claims 1 to 6, **characterized in that** the said polysaccharide is a substituted dextran.

12. Use according to claim 11, **characterized in that** the said polysaccharide is a CMDBS.

13. Use according to one of claims 1 to 5, **characterized in that** the said polymer is of non-osidic nature.

14. Use or composition according to one of claims 1 to 13, **characterized in that** the muscular tissue is that of cardiac or skeletal muscles.

15. Pharmaceutical composition for the healing of skeletal or cardiac muscular tissues containing at least one polymer such as defined in claims 1 to 11 in combinaison with at least one pharmacologically acceptable excipient, with the exception of a pharmaceutical composition comprising a combinaison of heparine and sulodexide.
